# FASCICULE DE BREVET EUROPEEN

(11) **EP 1 761 635 B1**
(45) Date de publication et mention de la délivrance du brevet: **14.09.2011**
(21) Numéro de dépôt: 05779704.5
(22) Date de dépôt: 24.06.2005
(51) Int. Cl.: C12N 15/867, C12N 5/10, A61K 35/76, A61K 39/21, A61K 48/00

(54) **LENTIVIRUS NON INTEGRATIF ET NON REPLICATIF, PREPARATION ET UTILISATIONS**
NICHT INTEGRATIVER UND NICHT REPLIKATIVER LENTIVIRUS, HERSTELLUNG UND VERWENDUNGEN
NON-INTEGRATIVE AND NON-REPLICATIVE LENTIVIRUS, PREPARATION AND USES THEREOF

(30) Priorité: 25.06.2004 FR 0407017
(43) Date de publication de la demande: 14.03.2007
(73) Titulaire: CENTRE NATIONAL DE LA RECHERCHE SCIENTIFIQUE, 75794 Paris Cedex 16 (FR)
(72) Inventeur: MALLET, Jacques, 75013 Paris (FR); SERGUERA, Che, I-00196 Rome (IT); PHILIPPE, Stéphanie, F-75010 Paris (FR); SARKIS, Chamsy, 92100 BOULOGNE BILLANCOURT (FR)
(74) Mandataire: Starck-Loudes, Anne-Caroline
(86) Numéro de dépôt international: PCT/FR2005/001604
(87) Numéro de publication internationale: WO 2006/010834

(56) Documents cités:
- WO-A-00/72886
- WO-A-97/31119
- LEAVITT A D ET AL: "Human immunodeficiency virus type 1 integrase mutants retain in vitro integrase activity yet fail to integrate viral DNA efficiently during infection" JOURNAL OF VIROLOGY, THE AMERICAN SOCIETY FOR MICROBIOLOGY, US, vol. 70, no. 2, février 1996 (1996-02), pages 721-728, XP002207365 ISSN: 0022-538X
- WISKERCHEN M ET AL: "HUMAN IMMUNODEFICIENCY VIRUS TYPE 1 INTEGRASE: EFFECTS OF MUTATIONSON VIRAL ABILITY TO INTEGRATE, DIRECT VIRAL GENE EXPRESSION FROM UNINTEGRATED VIRAL DNA TEMPLATES, AND SUSTAIN VIRAL PROPAGATION IN PRIMARY CELLS" JOURNAL OF VIROLOGY, THE AMERICAN SOCIETY FOR MICROBIOLOGY, US, vol. 69, no. 1, janvier 1995 (1995-01), pages 376-386, XP000560257 ISSN: 0022-538X
- FOLLENZI A ET AL: "Gene transfer by lentiviral vectors is limited by nuclear translocation and rescued by HIV-1 pol sequences" NATURE GENETICS, NATURE AMERICA, NEW YORK, US, vol. 25, no. 2, juin 2000 (2000-06), pages 217-222, XP002980776 ISSN: 1061-4036

## Description

La présente invention décrit un lentivirus recombinant non intégratif et non réplicatif ainsi que ses utilisations, notamment pour la préparation d'une composition destinée au transfert de gènes *in vitro*, *ex vivo* ou *in vivo.* L'invention est utilisable pour le transfert de gènes dans tout organisme mammifère, par exemple dans les tissus ou cellules du foie, muscle, pancréas et du système nerveux central (y compris de la sphère oculaire), et notamment pour le traitement de désordres et pathologies tels que par exemple des désordres du système nerveux central, y compris de la sphère oculaire.

Le transfert de gènes dans le système nerveux présente de multiples applications, notamment dans les domaines expérimentaux (e.g., recherche) et thérapeutiques. Ainsi, ce transfert peut permettre la réalisation d'études de marquage, de toxicité, de qualité, la construction de modèles pathologiques, la restauration de déficits, l'expression de produits (e.g., protéines, ARNs, etc.) thérapeutiques, etc.

Différentes approches ont été envisagées dans l'art antérieur pour ce transfert, telles que l'emploi de vecteurs viraux (rétrovirus, AAV, adénovirus, etc.), l'injection de plasmides, la greffe de cellules, l'implantation de cellules encapsulées, etc. Chacune de ces approches présente des avantages et des inconvénients, en terme d'efficacité, de sécurité, d'utilisation industrielle, de sélectivité, de stabilité, etc. Ainsi, l'utilisation de vecteurs viraux est avantageuse en terme d'efficacité de transfert, liée aux propriétés naturelles d'infection des virus.

Dans ce contexte, de nombreux vecteurs rétroviraux dérivés d'oncorétrovirus permettent l'intégration d'un transgène dans le génome de cellules cibles, mais ces vecteurs sont uniquement capables de transduire des cellules en division.

Cette restriction limite leur utilisation au transfert de gène *ex vivo* ou aux organes dont les cellules sont mitotiquement actives.

Afin de contourner cet obstacle, il a été envisagé d'utiliser des vecteurs dérivés de lentivirus. Par rapport aux autres vecteurs viraux couramment développés, les vecteurs lentiviraux présentent plusieurs avantages pratiques, dont la facilité de production à haut titre et une connaissance accrue de leur biologie. Ces vecteurs sont largement utilisés pour le transfert de gènes dans le cadre de protocoles de thérapie génique expérimentale. Ils permettent de transduire efficacement de nombreux types cellulaires, notamment les cellules quiescentes du système nerveux central. Les lentivirus sont en effet des rétrovirus complexes capables de s'intégrer dans le génome de cellules non mitotiquement actives. Des exemples de tels lentivirus sont les virus HIV-1, HIV-2, SIV, FIV, BIV, VISNA, CAEV et EIAV. Toutefois, un inconvénient lié à l'utilisation des rétrovirus, et notamment des lentivirus, réside en particulier dans le fait qu'ils présentent un risque potentiel de mutagenèse insertionnelle puisqu'ils s'intègrent dans la chromatine des cellules transduites et, de manière apparemment préférentielle, dans les séquences codantes. Cet inconvénient a jusqu'à présent limité l'exploitation de ce type de vecteur pour le transfert de gènes *in vivo.*

Dans le but de réduire le risque de mutagenèse insertionnelle, qui constitue à l'heure actuelle l'obstacle majeur à l'utilisation de ces vecteurs en clinique, les inventeurs ont développé un vecteur lentiviral non intégratif et non réplicatif. Le caractère non intégratif de cette nouvelle génération de vecteurs lentiviraux représente donc une avancée considérable en terme de biosécurité, en particulier pour la thérapie génique. Ce vecteur peut être utilisé par exemple pour l'expression stable d'un transgène ou d'autres acides nucléiques dans des cellules qui ne se divisent pas ou pour l'expression transitoire d'un gène dans des cellules en division réfractaires à d'autres méthodes de transfection ou même de transduction par d'autres vecteurs.

Les documents Leavitt et al. (Journal of Virology, Feb. 1996, p. 721-728) et Wiskerchen et al. (Journal of Virology, Jan. 1995, p. 376-386) décrivent des virus VIH-1 mutants non intégratifs et le document WO 97/31119 décrit des rétrovirus mutants non intégratifs. Toutefois, à la différence des lentivirus selon l'invention, utilisables en toute sécurité comme vecteur d'expression d'un transgène, aucun des virus décrits dans ces documents n'est assimilable à un vecteur d'expression d'un transgène. Le document Follenzi et al. (Nature Genetics, volume 5, Juin 2000) décrit des vecteurs lentiviraux permettant le transfert de gène mais ne décrit aucun vecteur non intégratif et ne fait référence à aucune mutation de l'intégrase.

Des vecteurs non intégratifs sont connus dans l'art antérieur, dont les plus communs sont les vecteurs adénoviraux et les vecteurs herpétiques. Cependant, pour ces types de vecteurs, il n'existe pas à l'heure actuelle de méthode de production permettant d'obtenir des lots exempts de tout contaminant réplicatif. Parmi les vecteurs intégratifs, les vecteurs AAV, bien qu'ils ne s'intègrent qu'avec une fréquence relativement faible (environ 10%), sont limités par la taille du transgène qui peut y être cloné ainsi que par les mutations qu'ils provoquent également au niveau de leur site d'insertion.

La présente invention apporte donc une solution aux problèmes de l'art antérieur et fournit de nouveaux outils et vecteurs pour le transfert de gènes dans le système nerveux.

L'invention réside plus particulièrement dans la mise au point d'un lentivirus recombinant non réplicatif et non intégratif.

Les lentivirus de l'invention comprennent, de manière générale, une intégrase mutée et un génome recombinant particulier. Plus préférentiellement, les lentivirus selon l'invention comprennent (i) un génome recombinant comprenant, entre les séquences LTR 5' et 3' lentivirales, une séquence psi d'encapsidation lentivirale, un élément d'export nucléaire de l'ARN, un transgène et, éventuellement, un promoteur et/ou une séquence favorisant l'import nucléaire de l'ARN, ainsi que (ii) une intégrase mutée empêchant l'intégration dudit génome dans le génome d'une cellule hôte. Dans un mode particulier de réalisation de l'invention, le génome recombinant comprend par exemple la séquence 5'LTR-psi-RRE-cPPT CTS-transgène-LTR3'.

Un autre objet de l'invention concerne toute composition pharmaceutique (y compris vaccinale) comprenant un lentivirus selon l'invention et un excipient acceptable sur le plan pharmaceutique.

L'invention concerne également des méthodes et compositions destinées au transfert *in vitro, ex vivo* et *in vivo* de gènes cibles dans des populations de cellules particulières, et également au traitement de désordres par exemple, de désordres du système nerveux central, y compris du système oculaire.

Un autre objet de l'invention se rapporte à l'utilisation d'un lentivirus non intégratif et non réplicatif tel que défini ci-dessus pour la préparation d'une composition destinée au transfert de gènes dans une cellule de mammifère (humain de préférence), de préférence dans une cellule du système nerveux central (y compris de la sphère oculaire) d'un sujet *in vitro*, *ex vivo* ou *in vivo.*

L'invention a aussi pour objet tout procédé de production d'un lentivirus non réplicatif et non intégratif tel que défini ci-dessus, comprenant notamment l'introduction d'un plasmide vecteur comprenant, dans des cellules, un génome recombinant tel que défini précédemment, en présence des fonctions de transcomplémentation appropriées, et notamment d'une région pol lentivirale codant une intégrase modifiée telle que définie ci-dessus. Le procédé peut être mis en oeuvre par transfection transitoire de différents plasmides de transcomplémentation et d'enveloppe, ou en présence de virus auxiliaire, et/ou dans des lignées cellulaires exprimant une ou plusieurs des protéines de complémentation.

Une lignée de cellules exprimant de manière stable une intégrase de préférence lentivirale comprenant une mutation induisant une perte de la fonction d'intégration de ladite intégrase est également décrite. La mutation, au sens de l'invention, peut correspondre à des mutations ponctuelles et/ou à des microdélétions de quelques bases de l'intégrase. Elle correspond de préférence à une ou plusieurs mutations ponctuelles affectant une région basique, la région C-terminale (par exemple une région basique de la région C-terminale) et/ou le site catalytique de l'intégrase, ladite intégrase mutée étant dénuée de fonction intégrative.

Un objet particulier de l'invention concerne ainsi un procédé de préparation d'un lentivirus recombinant non réplicatif et non intégratif comprenant la transfection d'une cellule à l'aide d'un système vectoriel lentiviral non intégratif et non réplicatif comprenant :
a) un plasmide de transcomplémentation, dépourvu de signal d'encapsidation psi et comprenant une séquence gag lentivirale et une séquence pol lentivirale mutée codant une intégrase non fonctionnelle pour l'intégration, ledit plasmide étant éventuellement délété des gènes accessoires tels que vif, nef, vpu et/ou vpr,
b) un plasmide d'enveloppe comportant une séquence Promoteur-env-PolyA, et
c) un plasmide vecteur lentiviral comportant un génome recombinant comprenant, entre les séquences LTR 5' et 3' lentivirales, une séquence psi d'encapsidation lentivirale, un élément d'export nucléaire de l'ARN, un transgène et éventuellement un promoteur et/ou une séquence favorisant l'import nucléaire de l'ARN, ainsi qu'une intégrase mutée empêchant l'intégration dudit génome dans le génome d'une cellule hôte, ledit vecteur étant dépourvu de toute séquence codante du lentivirus, et la récupération des lentivirus produits.

### Description détaillée de l'invention

La présente invention décrit des lentivirus recombinants non intégratifs et non réplicatifs permettant le transfert de gènes dans toute cellule de mammifères, en particulier dans les cellules humaines. Il peut s'agir de cellules en division ou de cellules quiescentes, de cellules appartenant à des organes centraux ou à des organes périphériques, tels que le foie, le pancréas, un muscle, le coeur, etc.. Un objet particulier de l'invention concerne le transfert de gènes dans le système nerveux (système oculaire compris) et notamment dans les neurones, les cellules gliales de type astrocytaire et les cellules rétiniennes, ainsi que dans les tumeurs cancéreuses. Ces vecteurs lentiviraux sont utiles pour le transfert et l'expression *in vivo* de séquences d'acides nucléiques en particulier au sein du système nerveux.

### Structure générale des vecteurs

Comme d'autres rétrovirus, les lentivirus possèdent des gènes *gag*, *pol* et *env* flanqués de deux séquences LTR (Long Terminal Repeat). Chacun de ces gènes code pour de nombreuses protéines qui sont initialement exprimées sous la forme d'un unique polypeptide précurseur. Le gène *gag* code pour les protéines de structure internes (capsides et nucléocapside). Le gène *pol* code pour la transcriptase inverse, l'intégrase et la protéase. Le gène *env* code pour la glycoprotéine d'enveloppe virale. Le génome des lentivirus contient en outre un élément RRE (Rev Responsive Element) agissant en cis responsable de l'export hors du noyau de l'ARN génomique viral qui sera encapsidé. Les séquences LTR 5' et 3' servent à promouvoir la transcription et la polyadénylation des ARN viraux. Le LTR contient toutes les autres séquences agissant en cis nécessaires à la réplication virale. Des séquences nécessaires à la transcription inverse du génome (site de liaison de l'amorce de l'ARNt) et à l'encapsidation de l'ARN viral dans des particules (site Ψ) sont adjacentes au LTR 5'. Si les séquences nécessaires à l'encapsidation (ou à l'empaquetage de l'ARN rétroviral dans les virions infectieux) sont absentes du génome viral, l'ARN génomique ne sera pas activement encapsidé. Le génome lentiviral comprend en outre des gènes accessoires tels que vif, vpr, vpu, nef, TAT, REV, etc.

La construction de vecteurs lentiviraux pour des applications de transfert de gènes a été décrite par exemple dans les brevets US 5,665,577, EP 386 882, US 5,981,276, US 6, 013, 516 ou encore dans les demandes de brevet WO99/58701 et WO02/097104. Ces vecteurs comportent un génome lentiviral défectif, c'est-à-dire dans lequel l'un au moins des gènes *gag*, *pol* et *env* a été inactivé ou délété.

Le vecteur lentiviral selon l'invention est un lentivirus recombinant non réplicatif et non intégratif, c'est-à-dire qu'il est incapable de réplication autonome et d'intégration spécifique dans les cellules transduites. Un lentivirus recombinant non réplicatif et non intégratif décrit dans le présente texte comprend un génome recombinant comprenant, entre les séquences LTR 5' et 3' lentivirales, une séquence psi d'encapsidation lentivirale, et un élément d'export nucléaire de l'ARN, un transgène et éventuellement un promoteur et/ou une séquence favorisant l'import nucléaire de l'ARN, ainsi qu'une intégrase mutée empêchant l'intégration dudit génome dans le génome d'une cellule hôte. Le lentivirus selon l'invention peut par exemple comprendre la séquence 5'LTR-psi-RRE-cPPT CTS-transgène-LTR3'.

Un lentivirus préféré selon l'invention est un lentivirus dont le génome est avantageusement dépourvu de toute séquence lentivirale codante.

Une caractéristique importante des lentivirus de l'invention réside dans le fait qu'ils comprennent une intégrase modifiée. La présente invention démontre, pour la première fois, qu'il est possible de produire, dans des conditions efficaces pour l'expression d'un transgène, des lentivirus recombinants non réplicatifs, et dont les propriétés d'intégration sont altérées. La présence d'une intégrase modifiée résulte de l'utilisation, pour produire les virus de l'invention, d'une séquence pol modifiée de manière à produire une intégrase non fonctionnelle pour l'intégration, mais sans effet substantiel sur les étapes précédentes du cycle du vecteur lors de la transduction cellulaire (mutation dite de classe 1). Le vecteur obtenu présente alors un phénotype épisomal dans la mesure où, avantageusement, la mutation de l'intégrase n'empêche pas la progression du génome vers le noyau, ni sa rétrotranscription sous la forme d'un génome ADN linéaire qui peut alors se circulariser (formation de cercles). Au sens de l'invention, une mutation de classe 1 consiste préférentiellement en une ou plusieurs mutations ponctuelles, affectant de préférence la portiond'acide nucléique codant une région basique, la région C-terminale (de préférence une région basique de la région C-terminale) et/ou le site catalytique de l'intégrase. La mutation ponctuelle se traduit de préférence par la susbtitution d'un acide aminé par un autre au niveau de la séquence codée de l'intégrase. La mutation est de préférence non conservative dans le sens où elle rend l'intégrase non fonctionnelle pour l'intégration. Une telle mutation est préférentiellement choisie parmi les mutants produisant une intégrase non fonctionnelle pour l'intégration, tout en conservant les autres fonctions de l'intégrase, e.g., celles participant à la progression du vecteur vers le noyau. Des exemples de mutations affectant HIV-1 et permettant l'obtention d'une intégrase non fonctionnelle pour l'intégration sont les suivants :
H12N, H12C, H16C, H16V, S81R, D41A, K42A, H51A, Q53C, D55V, D64E, D64V, E69A, K71A, E85A, E87A, D116N, D116I, D116A, N120G, N120I, N120E, E152G, E152A, D-35-E, K156E, K156A, E157A, K159E, K159A, K160A, R166A, D167A, E170A, H171A, K173A, K186Q, K186T ( région L de la région basique C terminale), K188T , E198A, R199C, R199T, R199A, D202A, K211 A, Q214L (214 et 216 appartiennent à la région Q de la région basique C terminale), Q216L, Q221 L, W235F, W235E, K236S, K236A, K246A, G247W, D253A, R262A, R263A (région N de la région basique C terminale) et K264H.

Les mutations affectant le site catalytique concernent de préférence, pour ce qui concerne HIV-1, les acides aminés 64, 116 et/ou 152 de l'intégrase. Les mutations affectant la portion C-terminale de ce lentivirus sont avantageusement choisies parmi la substitution du motif ₂₆₂RRK par AAH, une substitution dans la région Q (Q214L et/ou Q216L), dans la région L (K186) et/ou dans la région L. Une mutation préférée consiste en la substitution du motif ₂₆₂RRK par AAH.

Les lentivirus de l'invention comportent typiquement un génome recombinant de séquence 5'LTR-psi-RRE-cPPT CTS-(promoteur-)transgène-LTR3'. Le transgène est typiquement placé sous le contrôle d'un promoteur. L'un et/ou l'autre peuvent également être placé en amont de l'élément cPPT CTS.

Le génome recombinant comporte ainsi les séquences virales agissant en *cis*, utiles à l'encapsidation et à la transduction. Avantageusement, il ne conserve que certaines séquences lentivirales, notamment
- celles nécessaires à l'encapsidation du génome (séquence psi d'origine lentivirale) ;
- un élément d'export nucléaire de l'ARN. Celui-ci est avantageusement choisi parmi l'élément de réponse à REV (séquence RRE pour "REV Responsive Element") d'un génome de lentivirus, notamment d'un VIH, et par exemple du VIH-1 (la séquence RRE, présente sur l'ARN viral, interagit avec un élément de régulation REV), le CTE ("Constitutive Transport Element") du virus Mason Pfizer Monkey, un système d'export nucléaire du SIV, du HIV-2 ou du FIV, ou un élément équivalent de tout autre rétrovirus (par exemple le système d'export nucléaire du SIV, du HIV2 ou du FIV) ;
- une séquence favorisant l'import nucléaire de l'ARN, par exemple la séquence flap [région cPPT-CTS (central polypurine tract- central terminaison sequence) ; cf. Charneau et al., Journal of Virology, May 1992; WO 01/27304] qui permet un import nucléaire efficace du génome vecteur reverse-transcrit ; et
- un LTR 5' et 3', éventuellement modifié(s), intervenant dans la transcription de l'ARN vecteur encapsidé.

Le génome recombinant est préférentiellement délété de la totalité des séquences codantes lentivirales, en particulier des gènes viraux codant pour les séquences gag, pol et env et des gènes accessoires vif, vpr, vpu et nef. Le plasmide transcomplémentant conserve de préférence les gènes tat et rev. Le plasmide vecteur peut comporter avantageusement un LTR3' délété de la séquence enhancer U3 (WO99/31251) pour améliorer l'expression du transgène et la sécurité du vecteur.

Une autre cible peut éventuellement être mutée en complément des mutations d'intégrase : les séquences att qui se situent aux extrémités du génome linéaire ADN. Si ces séquences sont mutées, la prise en charge du génome par l'intégrase ne se fait plus correctement et il ne peut plus y avoir intégration. Le génome qui se présente sous la forme d'un ADN linéaire peut se circulariser.

Les vecteurs et plasmides de l'invention peuvent être préparés à partir de lentivirus appartenant à différentes espèces, notamment HIV-1, HIV-2, SIV, FIV, BIV, VISNA, CAEV et EIAV. Des sérotypes particulièrement préférés sont le HIV, notamment le HIV-1, FIV, EIAV et SIV.

La séquence du transgène peut être placée sous contrôle d'un promoteur et/ou d'un enhancer choisi, ainsi que de tous les éléments de régulation transcriptionnelle, post transcriptionnelle et post traductionnelle nécessaires à la bonne expression dudit transgène.

Au sens de l'invention, le terme « transgène » désigne, de manière générale, tout acide nucléique codant ou non. Il peut s'agir d'une séquence non codante telle que par exemple une séquence de reconnaissance d'un enzyme (site d'intégration spécifique, site présentant une affinité particulière pour une protéine, etc.). Il s'agit de préférence d'une séquence codant un polypeptide donné ou un ARN actif en tant que tel. Il peut s'agir notamment d'un ADNc, d'un ADNg, d'un ADN synthétique, d'un ARN, par exemple un ARN interférent, un ribozyme, etc., ou d'une combinaison de ceux-ci. Typiquement, le transgène est un ADN comprenant une séquence codant le produit d'expression désiré. Le transgène peut comporter en outre une ou des régions de terminaison de la transcription, typiquement un signal de polyadénylation.

Le transgène peut être choisi parmi un acide nucléique catalytique (interférent, antisens, ribozyme), un acide nucléique suicide (e.g., codant une toxine) ou un acide nucléique codant un peptide biologiquement actif, par exemple un facteur de croissance, un facteur trophique, un facteur anti-angiogénique, une hormone, une cytokine, un anticorps, un récepteur, un facteur de différentiation, un facteur de stimulation des colonies, un agent anticancéreux, un enzyme, un neurotransmetteur ou son précurseur, etc.

Selon un mode de réalisation particulier de l'invention, le transgène code par exemple pour les facteurs trophiques suivants: CNTF, NGF, NT3, NT4, FGF, PDGF, GDNF, etc., ou pour des facteurs anti-angiogéniques ou pour des enzymes restaurant une activité métabolique déficiente ou apportant une fonction métabolique particulière, par exemple: TH, AADC, GTPC, β-glucuronidase, etc.

Selon un autre mode de réalisation particulier de l'invention, le transgène code, par exemple, pour des ARN interférents (ARNi) permettant d'inhiber de manière spécifique l'expression de protéines mutées impliquées dans une maladie génétique dominante ou dans une maladie induite par un gain de fonction, par exemple une maladie neurodégénérative telles que la SOD mutée (Sclérose Latérale Amyotrophique), les protéines APP, tau, préséniline, ou BACE (maladie d'Alzheimer), l'α-synucléine (maladie de Parkinson) ou la Huntingtine (maladie de Huntington).

Le transgène est typiquement placé sous contrôle d'un promoteur transcriptionnel, qui peut être homologue vis-à-vis du transgène ou hétérologue, par exemple un promoteur cellulaire, viral, synthétique, chimérique, etc. Le promoteur utilisé peut être constitutif ou régulé, faible ou fort, spécifique de tissu ou ubiquitaire, dépendant de l'ARN polymérase 2 ou 3, etc. On utilise typiquement un promoteur viral tel que CMV, RSV LTR, TK, etc. ou de préférence un promoteur cellulaire tel que PGK, Rho, EF1α, etc. Des promoteurs spécifiques de tissus peuvent être employés. Il peut s'agir par exemple des promoteurs ENO, GFAP, NSE, d'un promoteur de l'ARN polymérase III tel que le promoteur U6 ou H1, éventuellement modifié, etc. Le promoteur utilisé pour diriger l'expression du transgène peut être par exemple un promoteur viral choisi parmi le promoteur du gène CMV, TK ou RSV LTR.

Le promoteur présent dans le plasmide d'enveloppe et/ou le promoteur présent dans le plasmide vecteur sont identiques ou différents et cellulaires ou viraux.

### Production de vecteurs lentiviraux non intégratifs et non réplicatifs

Les vecteurs lentiviraux selon l'invention peuvent être préparés de différentes manières, par transfection(s) transitoire(s), dans des lignées stables et/ou au moyen de virus helper.

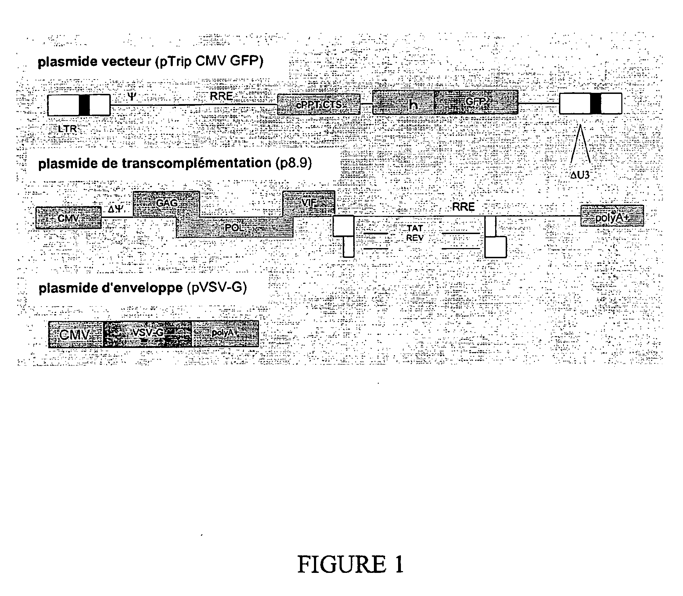

Le procédé prévoit, selon un mode particulièrement préféré, la combinaison d'un minimum de trois plasmides (cf. figure 1) pour produire un virion recombinant ou un lentivirus recombinant :
a) un plasmide de transcomplémentation, dépourvu de signal d'encapsidation psi et comprenant une séquence gag lentivirale et une séquence pol lentivirale mutée codant une intégrase non fonctionnelle pour l'intégration, ledit plasmide étant éventuellement délété des gènes accessoires vif, nef, vpu et/ou vpr,
b) un plasmide d'enveloppe comportant une séquence Promoteur-env-PolyA, et
c) un plasmide vecteur lentiviral comportant un génome recombinant, éventuellement délété de la région promotrice du LTR3' ou de la séquence enhancer U3 du LTR3', comportant, entre les séquences LTR 5' et 3' lentivirales, une séquence psi d'encapsidation lentivirale, un élément d'export nucléaire (de préférence le RRE du HIV ou un élément équivalent de tout autre rétrovirus) de l'ARN, un transgène et éventuellement un promoteur et/ou une séquence favorisant l'import nucléaire (séquence cPPT CTS par exemple) de l'ARN, ainsi qu'une intégrase mutée empêchant l'intégration dudit génome dans le génome d'une cellule hôte, ledit vecteur étant dépourvu de toute séquence codante du lentivirus, et
la récupération des lentivirus produits.

Avantageusement, les trois plasmides utilisés ne comportent pas de séquence homologue suffisante pour permettre une recombinaison. Les acides nucléiques codant *gag*, *pol* et *env* peuvent être avantageusement des ADNc préparés selon les techniques conventionnelles, à partir de séquences des gènes viraux disponibles dans l'art antérieur et sur bases de données, ainsi qu'illustré dans les exemples.

Le plasmide de trans-complémentation fournit un acide nucléique codant les protéines lentivirales gag et *pol.* Ces protéines sont dérivées d'un lentivirus et, de manière préférée, proviennent du VIH-1. Le plasmide est dépourvu de séquence d'encapsidation, de séquence codant pour une enveloppe, des gènes accessoires et, avantageusement, est également dépourvu de LTRs lentiviraux. De ce fait, les séquences codant pour des protéines *gag* et *pol* sont avantageusement placées sous contrôle d'un promoteur hétérologue, par exemple cellulaire, viral, etc., qui peut être constitutif ou régulé, faible ou fort. Il s'agit de préférence d'un plasmide transcomplémentant comportant une séquence CMV-Δpsi-gag-pol-PolyA. Ce plasmide permet l'expression de toutes les protéines nécessaires à la formation de virions vides, exceptées les glycoprotéines d'enveloppe. Le plasmide de transcomplémentation peut avantageusement comprendre les gènes TAT et REV. Le plasmide de transcomplémentation peut également comprendre en outre un élément de régulation de la transcription choisi parmi WPRE, le 5'UTR de l'APP, le 3'UTR de TAU et une séquence insulatrice de la chromatine telle que MAR (Matrix Attachment Region), SAR (Scaffold Attachement Region), scs et scs' (Special Chromatine Structure), etc.. Il est avantageusement dépourvu des gènes accessoires vif, vpr, vpu et/ou nef. Il est entendu que les gènes gag et pol, ainsi que les gènes TAT et REV, peuvent aussi être portés par des plasmides différents, éventuellement séparés. Dans ce cas, plusieurs plasmides de transcomplémentation sont utilisés, codant chacun pour une ou plusieurs desdites protéines.

La mutation dans la séquence pol du plasmide de transcomplémentation consiste en une ou plusieurs microdélétions de quelques bases, de préférence en une ou plusieurs mutations ponctuelles affectant une région basique, la région C-terminale (par exemple une région basique de la région C-terminale), et/ou la région catalytique de la séquence de l'intégrase codée, comme défini précédemment.

Le plasmide d'enveloppe fournit un acide nucléique qui permet la production de la glycoprotéine d'enveloppe (*env*) choisie. Il est dépourvu de signal d'encapsidation psi, de séquences codant gag ou pol et est également dépourvu de LTRs lentiviraux. Il comporte une séquence Promoteur-env-PolyA.

Des vecteurs VIH-1 pseudotypés (comportant une enveloppe différente de l'enveloppe sauvage, provenant par exemple d'un autre virus, ou, d'origine cellulaire, et possédant ainsi un tropisme modifié) décrits dans l'art antérieur comportent la glycoprotéine d'enveloppe du Virus de la Stomatite Vésiculaire (VSV). Cette enveloppe présente des caractéristiques avantageuses telles que la résistance à l'ultracentrifugation et un très large tropisme. Contrairement à d'autres enveloppes comme celles des rétrovirus classiques (les rétrovirus amphotrope et écotrope de MLV ou la gp120 du VIH mais aussi bien d'autres) la glycoprotéine de VSV n'est pas labile après ultracentrifugation. Ceci permet de concentrer les surnageants viraux et d'obtenir de hauts titres infectieux. Cette enveloppe confère par ailleurs aux virions un très large tropisme notamment *in vitro,* permettant l'infection de très nombreux types cellulaires. Le récepteur de cette enveloppe serait un motif phosphatidylsérine, présent à la surface de nombreuses cellules de différentes espèces.

La glycoprotéine d'enveloppe (env) du virus de la stomatite vésiculaire (VSV-G) est avantageusement utilisée dans le cadre de l'invention mais tout autre pseudotype peut être utilisé afin de cibler au mieux certaines populations cellulaires. La protéine d'enveloppe peut ainsi être choisie parmi toute glycoprotéine d'enveloppe de tout virus enveloppé, par exemple parmi une protéine d'enveloppe de rhabdovirus, plus préférentiellement de lyssavirus, encore plus préférentiellement d'un virus du sérogroupe du virus de la Rage: Rabies (RAB), Duvenhague (DUV), European bat type 1 (EB-1), European bat type 2 (EB-2), Kotonkan (KOT), Lagos bat (LB), Obodhiang (OBD), Rochambeau (RBU), une protéine d'enveloppe d'un virus du sérogroupe du virus de Mokola (MOK) et toute composition chimérique de ces enveloppes. Les virus de la rage et de Mokola sont particulièrement préférés. Ils ont en effet un tropisme chez l'animal très spécifique du système nerveux (cf. WO 02/097104). Ce type d'enveloppe permet en outre un ciblage cellulaire, notamment le ciblage des cellules gliales de type astrocytaire.

Dans un mode de réalisation préféré, l'invention utilise des vecteurs lentiviraux, par exemple de type VIH-1, pseudotypés avec une enveloppe de virus de la rage ou de Mokola.

Un plasmide vecteur comprend un acide nucléique recombinant comprenant entre les LTR 5' et 3', les éléments psi, RRE (ou un élément équivalent d'un autre rétrovirus), le transgène et éventuellement un promoteur et/ou la séquence flap, cPPT CTS. Il peut comporter par exemple la séquence 5'LTR-psi-RRE-cPPT CTS-(promoteur-)transgène-LTR3'. La séquence du transgène est éventuellement placée, au sein du plasmide vecteur, sous contrôle du promoteur et/ou d'un enhancer, ainsi que de tous les éléments de régulation transcriptionnelle, post transcriptionnelle et post traductionnelle nécessaires à la bonne expression de ce gène. Ce plasmide comporte les séquences virales agissant en *cis* et nécessaires au bon déroulement de la transduction. Il ne conserve du virus d'origine que certaines séquences nécessaires à l'encapsidation du génome (séquence psi d'origine lentivirale), éventuellement la séquence flap (région cPPT-CTS) qui permet un import nucléaire efficace du génome vecteur reverse-transcrit et un LTR 5' intègre qui permet la transcription de l'ARN vecteur devant être encapsidé. Ce vecteur peut en outre être éventuellement délété de la séquence enhancer U3 du LTR 3' (WO 99/31251). Il est par ailleurs délété de la totalité des gènes viraux d'origine, en particulier des gènes viraux codant pour les séquences gag, pol et env et des gènes accessoires (vif, nef, vpr et/ou vpu), pour améliorer la sécurité du vecteur.

Dans un mode de réalisation particulier, les séquences att qui se situent aux extrémités du génome linéaire sont en outre avantageusement mutées, éventuellement délétées pour gêner la prise en charge du génome par l'intégrase.

Les promoteurs utilisés dans le plasmide de transcomplémentation, le plasmide d'enveloppe et dans le plasmide vecteur pour promouvoir respectivement l'expression de gag et pol, de la protéine d'enveloppe, de l'ARNm du génome vecteur et du transgène sont des promoteurs identiques ou différents choisis avantageusement parmi des promoteurs ubiquitaires ou spécifiques, par exemple parmi les promoteurs viraux CMV, TK, RSV LTR et un promoteur de l'ARN polymérase III, tel que le promoteur U6 ou H1.

Les lentivirus selon l'invention sont modifiés génétiquement de manière à ce que certains gènes constitutifs du virus infectieux natif soient supprimés et remplacés par une séquence d'acide nucléique d'intérêt à introduire dans les cellules cibles. Après fusion du virus à la membrane cellulaire, celui-ci injecte son acide nucléique dans la cellule. Le matériel génétique ainsi transféré est ensuite transcrit et éventuellement traduit en protéines à l'intérieur de la cellule hôte.

Un système vectoriel préféré selon l'invention comprend :
a) un plasmide de transcomplémentation, dépourvu de signal d'encapsidation psi et comprenant une séquence gag lentivirale et une séquence pol lentivirale mutée codant une intégrase comportant une substitution du motif ₂₆₂RRK par AAH (dans une région basique de la région C-terminale de la séquence codée de l'intégrase), non fonctionnelle pour l'intégration, ledit plasmide étant dépourvu des gènes accessoires tels que vif, nef, vpu et/ou vpr,
b) un plasmide d'enveloppe tel que défini ci-dessus, comportant de préférence un promoteur viral et codant une enveloppe VSV-G, plus préférentiellement une séquence CMV-VSV-G-PolyA, et
c) un plasmide vecteur lentiviral, éventuellement délété de la région promotrice du LTR3' ou de la séquence enhancer U3 du LTR3', comportant entre les LTR 5' et 3', les éléments psi, RRE, le transgène et éventuellement un promoteur et/ou la séquence flap, cPPT CTS, ledit vecteur étant dépourvu de la totalité des gènes du lentivirus (par exemple HIV-1).

Pour la production des virus recombinants non intégratifs et défectifs pour la réplication, les plasmides décrits ci-avant peuvent être introduits dans des cellules compétentes et les virus fabriqués sont récoltés. Les cellules utilisées peuvent être toute cellule compétente, en particulier des cellules eucaryotes, notamment de mammifère, par exemple animale ou humaine. Elles peuvent être somatiques ou embryonnaires, souches ou différenciées. On peut citer par exemple les cellules 293, des cellules de fibroblastes, des hépatocytes, des cellules musculaires (squelettiques, cardiaques, lisses, vaisseau sanguin, etc.), nerveuses (neurones, gliales, astrocytes), des cellule épithéliales, rénales, oculaires, etc. Il peut également s'agir de cellules végétales, de levures ou de cellules procaryotes. Il peut aussi s'agir de cellules transformées à l'aide de l'antigène T du SV40.

L'invention réside donc dans un procédé de préparation d'un lentivirus non intégratif et non réplicatif recombinant, comprenant la transfection d'une population de cellules compétentes avec une combinaison de plasmides tels que décrits ci-avant, et la récupération des vecteurs produits.

Un procédé particulièrement avantageux de production de lentivirus non intégratifs et non réplicatifs permettant l'expression *in vivo* d'un transgène, comprenant la transfection de cellules compétentes à l'aide d'un système vectoriel lentiviral non intégratif et non réplicatif, tel que décrit ci-dessus, comprend :
a) au moins un plasmide de transcomplémentation, dépourvu de signal d'encapsidation psi et comprenant une séquence gag et/ou une séquence pol comportant une mutation de classe 1, permettant par exemple la substitution du motif ₂₆₂RRK par AAH dans une région basique et/ou dans la région C-terminale de la séquence codée de l'intégrase, ledit plasmide étant dépourvu des gènes accessoires tels que vif, nef, vpu et/ou vpr,
b) un plasmide d'enveloppe comportant une séquence promoteur (par exemple CMV)-enveloppe (par exemple VSV-G)-PolyA,
c) un plasmide vecteur, éventuellement délété de la séquence enhancer U3 du LTR3', comportant entre les LTR 5' et 3', les éléments psi, RRE, le transgène et éventuellement un promoteur et/ou la séquence flap, cPPT CTS, ledit vecteur étant délété des séquences codantes du lentivirus (par exemple HIV-1), et
la récupération des lentivirus produits.

Les lentivirus de l'invention peuvent également être préparés à partir de lignées de cellules d'encapsidation produisant une ou plusieurs protéines gag, *env* et/ou *pol* mutée au niveau de l'intégrase comme indiqué précédemment.

De ce fait, dans un mode particulier de mise en oeuvre, le procédé comprend la transfection de deux plasmides seulement (le plasmide vecteur et le plasmide de transcomplémentation) dans une lignée de cellules exprimant la protéine *env* choisie. Les cellules utilisées pour la préparation d'une telle lignée sont, par exemple, les cellules compétentes mentionnées ci-avant.

Selon un autre mode de réalisation, la lignée utilisée exprime également la protéine env, la protéine *gag* et/ou la protéine *pol* lentivirale, cette dernière comprenant une mutation de classe 1. Dans ce cas, le procédé comprend simplement la transfection du plasmide vecteur.

Comme indiqué précédemment, les lentivirus produits dérivent de manière préférée du virus VIH-1, VIH-2, SIV, FIV,BIV, VISNA, CAEV ou EIAV.

Parmi les lignées cellulaires on distingue notamment la lignée DT40 établie à partir d'un lymphome de poule connu pour être très recombinogène et la lignée Cos 7 (cellules de rein de singe immortalisée à l'aide d'un antigène SV40). Il peut également s'agir des lignées HCT116, DLD1 (lignées humaine établies à partir de cellules issues d'un carcinome colorectal), LF1 (fibroblastes de poumon embryonnaire humain), LL1 (fibroblastes de peau embryonnaire humaine), TK6 (lignée lymphoblastique humaine), HaCaT (kératinocytes humains), U937 (monocytes humains), HCT15, SW480, Colo320, Co115, EB, Hbl100, Rat-1, PC12 (photochromocytome de rat), etc. Cette liste non exhaustive est donnée à titre d'exemple. D'autres lignées connues de l'homme du métier peuvent être choisies et utilisées dans le cadre de l'invention sans effort particulier de sa part.

Pour la mise en oeuvre des procédés de l'invention, les plasmides peuvent être introduits dans les cellules par toute technique connue de l'homme du métier, adaptée au type cellulaire considéré. Généralement, les cellules et le système vectoriel sont mis en contact dans un dispositif approprié (plaque, boite, tube, poche, etc.), pendant une période de temps suffisante pour permettre le transfert du système vectoriel ou du plasmide dans les cellules. Typiquement, le système vectoriel ou le plasmide est introduit dans les cellules par précipitation au phosphate de calcium, par électroporation ou en utilisant un ou des composés facilitant la transfection, tels que des lipides, polymères, liposomes et peptides, etc. La précipitation au phosphate de calcium est préférée. Les cellules sont cultivées dans tout milieu adapté, tel que RPMI, DMEM, un milieu spécifique permettant une culture en l'absence de sérum de veau foetal, etc.

Une lignée de cellules est également décrite ainsi que l'utilisation d'une telle lignée de cellules pour la préparation *in vitro* de lentivirus recombinants non intégratifs et non réplicatifs. La lignée exprime de manière stable une intégrase lentivirale comprenant une ou plusieurs mutations ponctuelles affectant une région basique, sa région C-terminale (par exemple, une région basique de la région C-terminale) et/ou son site catalytique, ladite intégrase étant dénuée de fonction intégratives.

Les cellules obtenues par la mise en oeuvre du procédé et l'utilisation d'une cellule, lignée ou population cellulaire selon l'invention pour la préparation d'une composition cellulaire destinée à la mise en oeuvre d'une méthode de traitement thérapeutique, vaccinale ou chirurgicale chez l'homme ou l'animal sont ainsi également décrites.

Des kits pour la mise en oeuvre de procédés de modification du génome de cellules *in vitro* ou *ex vivo*, comprenant un vecteur tel que décrit ci-avant sont par ailleurs décrits.

### APPLICATIONS

Les virus et lignées selon l'invention peuvent être utilisés par exemple pour l'expression d'un transgène ou d'autres acides nucléiques préférentiellement dans des cellules qui ne se divisent pas ou pour l'expression transitoire d'un gène dans des cellules en division réfractaires à d'autre méthode de transfection ou même de transduction par d'autres vecteurs.

De manière surprenante, la présente demande montre que les vecteurs lentiviraux ainsi obtenus sont capables de transduire différents types cellulaires tels que, par exemple, des cellules rétiniennes, des astrocytes, d'autres cellules gliales ou des neurones. D'autres sous-populations de cellules nerveuses qui peuvent être ciblées par des vecteurs de l'invention sont par exemple des cellules microgliales, des cellules endothéliales ou des oligodendrocytes. Dans une application particulière concernant le transfert de gènes dans l'oeil, les vecteurs de l'invention peuvent être par exemple pseudotypés avec l'enveloppe Mokola afin de permettre un transfert sélectif vers les cellules de l'épithélium pigmentaire.

Ce vecteur lentiviral non intégratif et non réplicatif est destiné à améliorer la sécurité et l'efficacité du transfert de gènes : par la mutation de l'intégrase, le vecteur ne s'intègre plus dans le génome de la cellule cible, éliminant ainsi le risque de mutagenèse insertionnelle. Par ailleurs, l'insertion éventuelle de la séquence flap (cPPT-CTS) dans le vecteur, peut améliorer de façon substantielle l'import nucléaire du génome ADN, permettant une expression forte du transgène, stable dans les cellules post-mitotiques et transitoire dans les cellules se multipliant.

Les applications possibles des vecteurs lentiviraux non intégratifs de l'invention sont de plusieurs types et incluent :
- la thérapie génique, i.e., le transfert de gène dans toute cellule de mammifères, en particulier dans les cellules humaines. Il peut s'agir de cellules en division ou de cellules quiescentes, de cellules appartenant à des organes centraux ou à des organes périphériques, tels que le foie, le pancréas, un muscle, le coeur, etc. Il s'agit de préférence d'un transfert de gènes dans des cellules quiescentes (qui ne se divisent pas), notamment dans des cellules du système nerveux central, en particulier du cerveau, de la moelle et de la sphère oculaire, par exemple dans le cadre du traitement de pathologies neurodégénératives ou des atteintes de la rétine, et, d'un transfert de gène dans des cellules en division pour une expression transitoire (ex : stratégie suicide anti-tumorale, stratégie de repousse axonale pour traiter les traumas de la moelle épinière).

La thérapie génique peut permettre l'expression de protéines, par exemple de facteurs neurotrophiques, d'enzymes, de facteurs de transcription, de récepteurs, etc. Elle permet par ailleurs de mettre en oeuvre une stratégie « oligonucléotide » (ARN antisens ou interférents, ribozymes, etc.),
- la thérapie cellulaire, i.e., l'expression de facteurs de différenciation dans des cellules progénitrices pour orienter la cellule vers un destin choisi avant greffe ou la transduction *ex vivo* de cellules pour qu'elles expriment un facteur d'intérêt, suivie de la greffe desdites cellules.

Un objet particulier de l'invention concerne l'utilisation d'un lentivirus non intégratif et non réplicatif selon l'invention pour la préparation d'une composition destinée au transfert de gènes par exemple dans le système nerveux central (y compris la sphère oculaire) d'un sujet *in vitro*, *ex vivo* ou *in vivo*.

Un autre objet particulier de l'invention concerne l'utilisation d'un tel lentivirus pour la préparation d'une composition destinée au traitement d'une maladie affectant un organe central ou périphérique, par exemple d'une maladie du système nerveux (y compris de la sphère oculaire).

Selon le transgène qu'ils contiennent, les lentivirus non intégratifs et non réplicatifs selon l'invention peuvent être utilisés pour la fabrication d'une composition pharmaceutique destinée à traiter par exemple une maladie neurodégénérative et notamment la maladie d'Alzheimer, la maladie de Parkinson, la maladie de Huntington, les SLA ou SMA, les Dégénérescences maculaires liées à l'age (DMLA), des dégénérescences oculaires ou encore les traumas du système nerveux central (attaque cérébrale, épilepsie, lésions ou trauma de la moelle épinière, etc.), les maladies affectant le système nerveux central (mucopolysaccharidoses, etc.), les glioblastomes ou astrocytomes, les maladies métaboliques affectant le système nerveux (mucopolysaccharidoses, Charcot-Marie, etc.) ou des maladies affectant la sphère oculaire (DMLA, rétinites pigmentaires, glaucome, etc.).

Selon un mode de réalisation particulier de l'invention, les lentivirus recombinants non intégratifs et non réplicatifs sont utilisés pour la fabrication d'une composition pharmaceutique destinée à traiter la rétinite pigmentaire. Le terme de « rétinite pigmentaire » est un terme utilisé pour désigner un groupe hétérogène de désordres oculaires caractérisés par une dégénérescence progressive des bâtonnets et des cônes (cellules nerveuses de la rétine) par apoptose. Avec une incidence de 1 individu sur 3000, il s'agit de la cause majeure de cécité. La transduction des cellules de l'épithélium pigmentaire et des photorécepteurs présente un intérêt crucial dans ce type de pathologies. Une stratégie de remplacement génique nécessite la transduction des photorécepteurs ou de l'épithélium pigmentaire, alors qu'une stratégie de neuroprotection pourrait bénéficier de la transduction de l'épithélium pigmentaire. En effet, cette voie présente l'intérêt de ne pas modifier les cellules nerveuses mais uniquement l'épithélium pigmentaire qui synthétisera alors un facteur trophique diffusible, tel que le GDNF, et le sécrétera dans l'environnement des photorécepteurs à protéger.

Un autre objet ici décrit réside dans l'utilisation combinée de plusieurs lentivirus, en vue de transférer et d'exprimer plusieurs acides nucléiques dans les cellules du système nerveux. L'utilisation combinée peut comprendre des administrations séquentielles des différents virus, ou une administration simultanée.

Comme indiqué ci-avant, le transport et l'expression de multiples acides nucléiques dans les cellules nerveuses, comme par exemple des acides nucléiques catalytiques (interférents, antisens, ribozymes, etc.), des acides nucléiques codant des facteurs de croissance, des facteurs trophiques, des cytokines, des facteurs de stimulation des colonies, des agents anticancéreux, des toxines, des enzymes, des neurotransmetteurs ou leurs précurseurs, etc., sont possibles.

La composition pharmaceutique contenant le lentivirus selon l'invention peut être administrée à un patient par voie intracérébrale ou systémique compte-tenu du tropisme particulier des vecteurs lentiviraux pseudotypés à l'aide d'une glycoprotéine d'enveloppe appropriée. Ainsi, il peut s'agir d'une administration par voie intracérébrale par exemple intra-striatale, dans l'hypocampe ou la substance noire, par voie intra-veineuse, intra-artérielle, intra-vitrée, dans l'espace sous-rétinien, etc. Des modes d'injection préférés sont l'injection intracérébrale et l'injection dans l'espace sous-rétinien.

La composition est administrée avantageusement à raison de 10² à 10¹⁰, typiquement de 10³ à 10⁸, particules efficaces pour la transduction (titre déterminé par transduction de cellules par des dilutions en série du stock de vecteur) ou, en équivalent génome, de l'ordre de 10⁵ à 10¹³ copies [titre déterminé par reverse transcription-PCR (polymerase chain reaction) quantitative sur le génome ARN du vecteur ou par PCR quantitative sur le brin d'ADN associé au génome ARN du vecteur]. Les lentivirus peuvent être conditionnés dans toute solution adaptée, telle qu'une solution saline, isotonique, tamponnée, éventuellement associée à des agents stabilisants tels que de l'albumine isogénique ou tout autre protéine stabilisante, du glycérol, etc., ainsi que des facteurs adjuvants comme le polybrène ou le DEAE dextran, etc.

D'autres avantages de l'invention sont illustrés plus en détails dans les exemples qui suivent, qui doivent être considérés comme illustratifs et non limitatifs.

### LEGENDES DES FIGURES

Figure 1 :
   Système de production de vecteurs lentiviraux par transfection de trois plasmides : un plasmide vecteur portant le transgène GFP sous contrôle du promoteur du cytomégalovirus humain (hCMV) ainsi que la séquence du flap central [central polypurine tract- central terminaison sequence (cPPT-CTS)] qui intervient dans l'import nucléaire, la séquence RRE (REV Responsive Element) qui interagit avec un élément de régulation REV et la séquence d'encapsidation psi (ψ), la région U3 du LTR 3' a été délétée de la séquence promotrice (ΔU3) ; un plasmide de transcomplémentation exprimant les protéines nécessaires aux phases précoces du cycle réplicatif du VIH-1 (GAG et POL), des éléments de régulation (TAT, REV) sous contrôle du promoteur CMV et délété de la séquence ψ ; un plasmide d'enveloppe exprimant la glycoprotéine d'enveloppe du virus de la stomatite vésiculaire (VSV-G) sous contrôle du promoteur CMV.
Figure 2 :
   Expression de la GFP obtenue après transduction de lignées cellulaires (293T et HeLa) par un vecteur lentiviral non intégratif. Le pourcentage de transduction est déterminé par FACS 72h après incubation des cellules en présence de différentes doses (volume en microlitre par puit) d'un vecteur lentiviral intégratif IN_{WT} CMV GFP et non intégratif IN_{N} CMV GFP.
Figure 3 :
   Inhibition de la transduction par un vecteur lentiviral intégratif IN_{WT} CMV GFP et non intégratif IN_{N} CMV GFP après traitement des cellules à l'AZT. Le pourcentage de transduction a été déterminé par FACS après 72h d'incubation des cellules 293T en présence de vecteur seul ou de vecteur et d'AZT 10µM.
Figure 4 :
   Expression de la GFP au cours du temps après transduction de lignées cellulaires (293T et MT4) avec des vecteurs lentiviraux intégratif IN_{WT} CMV GFP ou non intégratif IN_{N} CMV GFP. Le pourcentage de cellules GFP⁺ a été déterminé par FACS (**A :** 293T, **B :** MT4) 3 jours, 6 jours, 9 jours et 12 ou 15 jours après transduction (MOI 5). Pour les points notés "but" : les cellules ont subit un traitement au butyrate de sodium 5mM 24h avant l'analyse.
Figure 5 :
   Expression de la GFP dans des neurones embryonnaires primaires issus de cortex de rats après transduction par un vecteur lentiviral intégratif IN_{WT} CMV GFP ou non intégratif IN CMV GFP
   A : Analyse immunocytochimique de l'expression dans des neurones contrôles (non transduits), transduits par le vecteur intégratif (IN_{WT}) ou transduits par le vecteur non intégratif (IN_{N}) 3, 9 et 16 jours après transduction (grossissement x20).
   B : pourcentage de transduction des neurones corticaux dans les groupes IN_{WT} et IN_{N}. Les mesures ont été réalisées en triplicate et exprimées en moyennes ± l'erreur standard (SEM).
   C : Analyse immunocytofluorescente de l'expression dans des neurones contrôles (non transduits), transduits par le vecteur intégratif (IN_{WT}) ou transduits par le vecteur non intégratif (IN_{N}) 3, 15 et 25 jours après transduction (grossissement x10).
Figure 6 :
   Expression de la GFP *in vivo* après injection dans le striatum de souris. Le cerveau a été prélevé 10 jours après injection stéréotaxique du vecteur IN_{N} CMV GFP (A) ou IN_{WT} CMV GFP (B) dans le striatum de souris, coupé au cryostat en coupe de 20µm d'épaisseur et analysé après immunohistochimie pour révéler la présence de GFP. cc : corps calleux, str : striatum. Le grossissement (x2.5, x5 ou x20) est indiqué sur chaque photographie.
Figure 7 :
   Expression de la GFP *in vivo* après injection dans le striatum de souris. Le cerveau a été prélevé 10 jours après injection stéréotaxique du vecteur INN CMV GFP dans le striatum de souris, coupé au cryostat en coupe de 20µm d'épaisseur et analysé après immunohistofluorescence. au microscope confocal.
   A : Comarquage GFP/GFAP : astrocyte exprimant la GFP (grossissement x40).
   B : Comarquage GFP/NeuN : neurone exprimant la GFP (grossissement x16).
Figure 8 :
   A : Expression de la GFP *in vivo* chez le rat après injection sous rétinienne de 66ng de p24 de vecteur lentiviral non intégratif INN CMV GFP. Photographie en microscopie fluorescence (x2.5) sur rétine montée à plat 2 semaines après injection.
   B : Expression de la GFP *in vivo* chez le chien après injection sous rétinienne de 2.5µg de p24 de vecteur lentiviral non intégratif INN CMV GFP. Angiographie en lumière fluorescente sur chien vigil 1 mois après injection.

### PARTIE EXPERIMENTALE

Dans un premier temps, les séquences d'intégrase (fusionnée à l'hémagglutinine), mutée ou non mutée dans le plasmide de transcomplémentation utilisé pour la production des vecteurs (plasmide p8.91 INWT et p8.91 INN) lentiviraux, ont été utilisées. Des stocks de vecteurs dérivés de VIH-1 exprimant la green fluorescent protein (GFP) sous contrôle du promoteur viral précoce du cytomegalovirus humain (hCMV) et portant l'intégrase normale (vecteur INWT CMV GFP) ou mutée (INN CMV GFP) ont ensuite été produits. Une étude portant sur l'efficacité des vecteurs INN CMV GFP pour diriger l'expression du transgène GFP au sein des cellules nerveuses a enfin été réalisée d'abord *in vitro* puis *in vivo.*

### Caractérisation du vecteur in vitro

### ■ Expression du transgène GFP in vitro

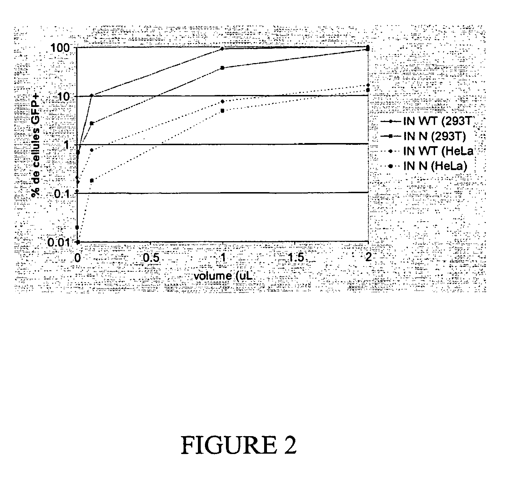

Afin d'évaluer la capacité d'un vecteur lentiviral non intégratif à transduire des lignées cellulaires et y exprimer un transgène, des cellules 293T, HeLa et MT4 ont été incubées en présence de différents volumes de vecteurs INWT CMV GFP et INN CMV GFP. Soixante douze heures après transduction par le vecteur intégratif INWT CMV GFP, un certain pourcentage de cellules expriment la GFP (analyse par FACS). De la même manière, des cellules GFP+ ont été mises en évidence par FACS 72 heures après transduction par le vecteur INN CMV GFP (figure 2). Ce premier résultat suggère une bonne efficacité de transduction des vecteurs dont l'intégrase a été inactivée.

### ■ Analyse de la pseudotransduction

Au cours de leur production, les stocks de vecteurs lentiviraux sont contaminés par de l'ADN plasmidique, notamment par le plasmide vecteur pTrip CMV GFP, ainsi que par la protéine GFP produite à partir de ce même plasmide dans les cellules transfectées. Ces deux éléments peuvent générer des cellules faussement positives, dans lesquelles la GFP n'est pas issue de l'expression du génome vecteur rétrotranscrit. Ces cellules GFP+ ne sont donc pas transduites mais "pseudotransduites".

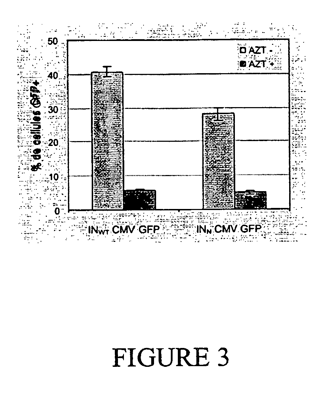

Afin de démontrer que les cellules GFP+ ne résultent pas d'un mécanisme de pseudotransduction, des cellules 293T ont été transduites, en présence ou en absence d'azido-deoxythymidine (AZT), à l'aide d'un inhibiteur de la reverse-transcriptase (RT). En effet, le traitement par l'AZT inhibe l'expression de la GFP uniquement si celle-ci résulte de la transduction des cellules. Le pourcentage de cellules GFP+ observées en présence d'AZT correspond donc au pourcentage de pseudotransduction. Après transduction de cellules 293T à une multiplicité d'infection (MOI) de 20, on observe 40,7% (± 1,5) de cellules GFP+ avec le vecteur intégratif INWT CMV GFP et 28,0% (± 1,9) avec le vecteur non intégratif INN CMV GFP. En présence de 10µM d'AZT, le pourcentage de cellules transduites tombe respectivement à 5,8% (± 0,1) et 4,9% (± 0,4) (figure 3). L'inhibition de la RT permet donc de réduire le pourcentage de cellules GFP+, que ce soit après transduction par le vecteur intégratif INWT CMV GFP ou par le vecteur INN CMV GFP. La plupart des cellules GFP+ observées en absence de l'inhibiteur de la reverse-transcriptase sont donc transduites efficacement par les deux types de vecteurs et ne résultent pas d'un mécanisme de pseudotransduction.

### ■ Stabilité de l'expression dans des cellules en division

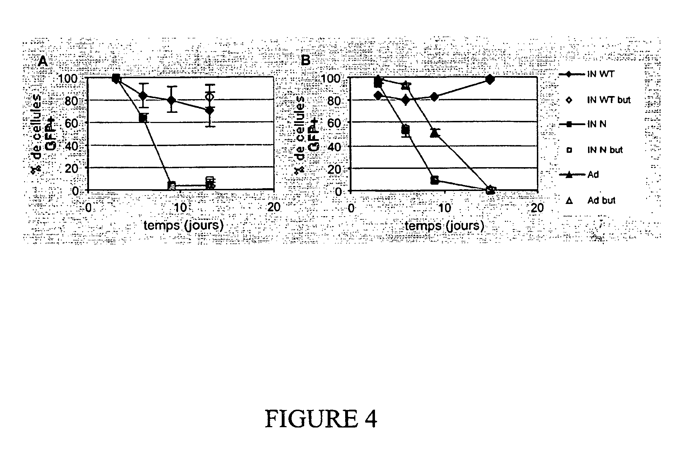

Pour vérifier le caractère épisomal des particules portant l'intégrase mutée IN-HAN, l'expression de la GFP a été analysée jusqu'à 15 jours après transduction dans les différentes lignées cellulaires HeLa, 293T et MT4. Les cellules ont été transduites à une MOI de 5 par le vecteur mutant INN CMV GFP, par le témoin intégratif INWT CMV GFP ou par un adénovirus CMV GFP, témoin non intégratif. Chaque lot de cellules transduites a été amplifié pendant 12 à 15 jours et analysé par FACS toutes les 72 heures. Vingt quatre heures avant de récolter les dernières cellules, une partie de celles-ci a été traitée au butyrate de sodium 5 mM afin d'évaluer l'influence éventuelle d'une hypoacétylation sur l'expression de la GFP. Les résultats obtenus sont présentés à la figure 4.

Au cours du temps, le pourcentage de cellules, 293T ou MT4, exprimant la GFP est relativement stable lorsque celles-ci sont transduites avec le vecteur témoin intégratif INWT CMV GFP, ce pourcentage diminuant légèrement aux derniers points évalués dans les cellules 293T (figure 4). Cependant, le traitement des cellules au butyrate de sodium à la fin de l'expérience permet de ramener le pourcentage de cellules 293T GFP+ au niveau initialement mesuré, ce qui suggère une réactivation du promoteur gouvernant l'expression du transgène et montre la stabilité dans le temps du vecteur intégré dans la population de cellules analysée.

Dans des cellules transduites avec un vecteur adénoviral, témoin non intégratif, le pourcentage de cellules positives chute significativement au cours des divisions successives pour s'annuler 15 jours après transduction. Dans ce cas, le traitement au butyrate de sodium à 15 jours après transduction ne permet pas de rétablir le pourcentage initial de cellules GFP+ (figure 4B). Ce résultat s'explique par la dilution progressive des génomes adénoviraux au cours des divisions cellulaires. En tant qu'éléments extrachromosomiques, les génomes vecteurs ne sont pas répliqués comme l'ADN génomique de la cellule pendant les cycles cellulaires. A chaque mitose, une copie du génome vecteur n'est donc transmise qu'à une seule des deux cellules filles, ce qui divise théoriquement le pourcentage de cellules exprimant le transgène par 2 à chaque cycle cellulaire.

L'expression dans les cellules transduites par le vecteur portant une intégrase mutante montre un profil proche de celui observé après transduction avec un vecteur adénoviral. En effet, le pourcentage de cellules GFP+ diminue au cours du temps (figure 4) et ne peut être ramené au niveau initial par un traitement des cellules au butyrate de sodium. Ce résultat suggère que, comme dans le cas d'une transduction par un vecteur adénoviral, le génome des vecteurs INN CMV GFP est éliminé des cellules initialement transduites par dilution successive à chaque division cellulaire.

### ■ Stabilité de l'expression du transgène GFP dans des cellules quiescentes

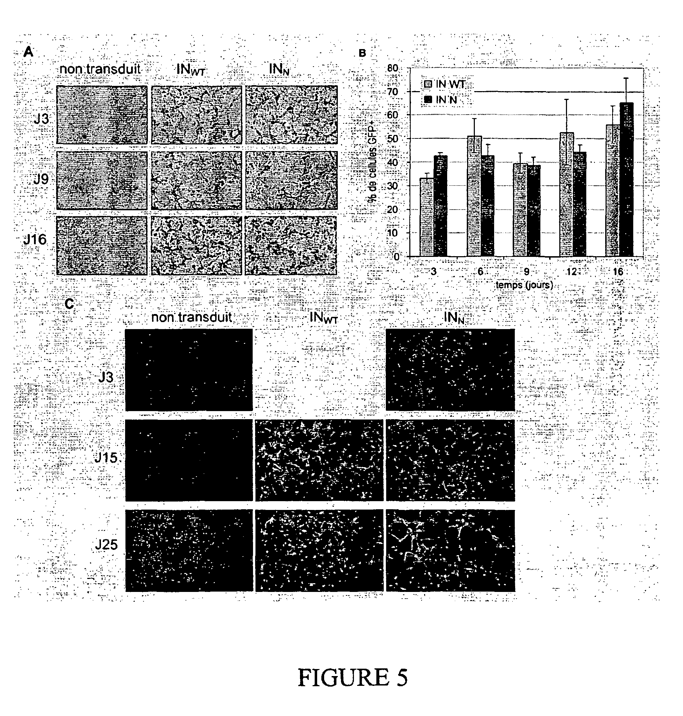

La réduction du pourcentage de cellules GFP+ au cours du temps pourrait également refléter l'instabilité des génomes vecteurs et leur dégradation dans le noyau des cellules transduites. Pour vérifier cette hypothèse, l'expression du transgène GFP a été étudiée après transduction de cellules neuronales par les deux types de vecteurs. En effet, les neurones primaires (cortex de rats embryonnaires) ne se divisent pas en culture. L'expression de la GFP persiste au moins 16 jours après transduction de ces cellules avec le vecteur INN CMV GFP (figure 5A et 5B). Aucune différence significative n'a pu être observée entre le pourcentage de cellules immunoréactives transduites par le vecteur WT aux différents temps considérés et celui obtenu avec le vecteur N (ANOVA 2 facteurs en mesures répétées, p = 0.9321. Une expérience supplémentaire a permis de montrer que l'expression du transgène persiste jusqu'à 25 jours après transduction (figure 5C).

Les formes épisomales sont donc relativement stables dans le noyau des cellules transduites et permettent une expression du transgène au moins pendant 25 jours dans des cellules quiescentes. Ce résultat conforte l'hypothèse d'une baisse de l'expression de la GFP au cours du temps dans des cellules en division par dilution des génomes vecteurs épisomaux à chaque mitose, plutôt que par dégradation.

### Expression du transgène GFP in vivo

### ■ Expression dans le striatum de souris

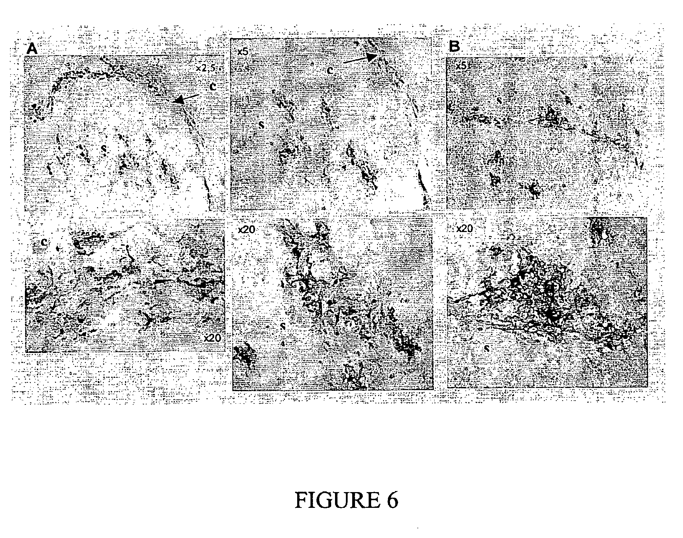

Afin de déterminer l'efficacité des vecteurs lentiviraux déficients pour l'intégrase à transduire des cellules *in vivo* et permettre l'expression d'un transgène GFP, le vecteur INN CMV GFP a été injecté dans le striatum de souris. Dix jours après cette injection, l'expression de la GFP a pu être mise en évidence. Cette expression perdure pendant un délai d'au moins un mois suivant l'injection. Ce résultat atteste de l'efficacité des vecteurs non intégratifs à permettre et à maintenir l'expression d'un transgène dans des cellules du SNC (figure 6).

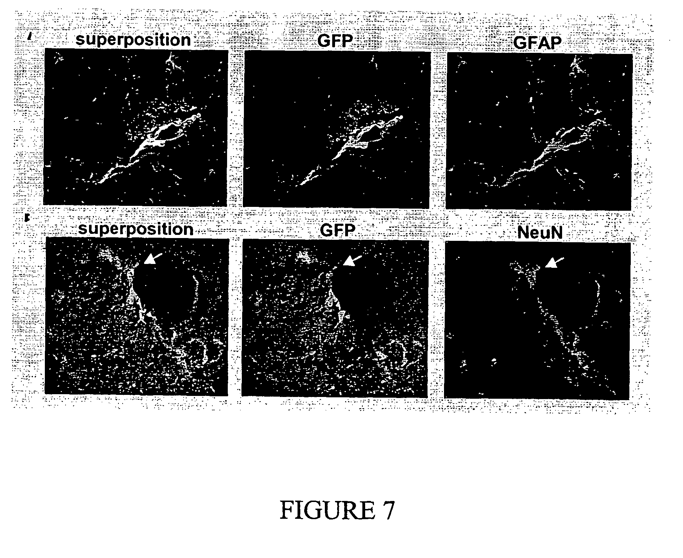

Afin d'identifier le phénotype des cellules transduites, un co-marquage en immunohistofluorescence GFP/GFAP (glial fibrillary acidic protein, marqueur des astrocytes) d'une part et GFP/NeuN (marqueur des neurones) d'autre part a été réalisé sur des coupes adjacentes. Après analyse des lames au microscope confocal, une colocalisation majoritaire de la GFP avec le marqueur GFAP (figure 7A) et très peu de colocalisation de la GFP avec le marqueur NeuN (figure 7B) ont pu être obervées. Ces résultats suggèrent que, *in vivo,* le vecteur INN CMV GFP pseudotypé avec l'enveloppe VSV, transduit préférentiellement des cellules astrocytaires.

### ■ Expression dans la rétine

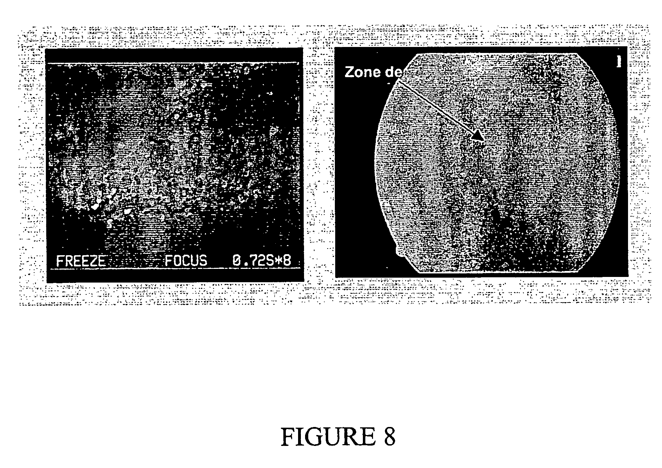

La capacité du vecteur lentiviral non intégratif à transduire les cellules de l'épithélium pigmentaire de la rétine a été évaluée. Pour cela, des injections sous rétiniennes du vecteur ont été réalisées chez des rats ainsi que chez des chiens. Les résultats préliminaires montrent une expression de la GFP pendant au moins 9 semaines chez le rat et 3 mois chez le chien (figure 8). L'évaluation de la stabilité de l'expression dans ces deux systèmes se poursuit. Des résultats obtenus précédemment par les inventeurs montrent que les vecteurs lentiviraux transduisent essentiellement, après injection sous rétinienne chez le rat, les cellules de l'épithélium pigmentaire.

Une faible toxicité a été observée chez quelques chiens par examen du fond d'oeil. Cette toxicité est indépendante de la dose injectée, donc du vecteur lui-même et semble induite par le geste chirurgical lors de l'injection. Une analyse histologique plus poussée permettra de rendre compte plus précisément de cette irritation.

### Expression d'un transgène thérapeutique dans un modèle animal

Dans le but de valider l'utilisation d'un vecteur lentiviral non intégratif pour une application clinique, l'efficacité dudit vecteur à exprimer un transgène thérapeutique, le Glial-derived neurotrophic factor (GDNF), a été testée après injection sous rétinienne chez le rat RD10, modèle caractérisé de rétinite pigmentaire.

L'expression « rétinite pigmentaire (RP) » désigne un groupe hétérogène de désordres oculaires caractérisés par une dégénérescence progressive, par apoptose, des bâtonnets et des cônes (cellules nerveuses de la rétine). Avec une incidence de un individu sur 3000, il s'agit de la cause principale de cécité. La transduction des cellules de l'épithélium pigmentaire et des photorécepteurs présente un intérêt crucial dans ce type de pathologies. Une stratégie de remplacement génique nécessite la transduction des photorécepteurs ou de l'épithélium pigmentaire, alors qu'une stratégie de neuroprotection pourrait bénéficier de la transduction de l'épithélium pigmentaire. En effet, cette voie présente l'intérêt de ne pas modifier les cellules nerveuses mais uniquement l'épithélium pigmentaire qui synthétisera alors un facteur neurotrophique diffusible, tel que le GDNF, et le sécrétera dans l'environnement des photorécepteurs à protéger.

Les expériences menées au laboratoire montrent que les vecteurs lentiviraux intégratifs sont efficaces pour la transduction des cellules de l'épithélium pigmentaire par injection sous rétinienne chez la souris normale.

Les résultats indiqués ci-dessus montrent l'efficacité et le potentiel thérapeutique des vecteurs lentiviraux non intégratifs selon l'invention capables de permettre l'expression d'un transgène *in vitro, ex vivo* et *in vivo* dans le système nerveux central (cerveau et rétine), chez le rongeur ainsi que chez le gros animal.

## Revendications

1. Lentivirus recombinant non réplicatif et non intégratif comprenant (i) un génome recombinant, dépourvu de toute séquence lentivirale codante, comprenant, entre les séquences LTR 5' et 3' lentivirales, une séquence psi d'encapsidation lentivirale, un élément d'export nucléaire de l'ARN, un transgène et, éventuellement, un promoteur et/ou une séquence favorisant l'import nucléaire de l'ARN, ainsi que (ii) une intégrase mutée empêchant l'intégration dudit génome dans le génome d'une cellule hôte, ladite mutation de l'intégrase consistant en une ou plusieurs mutations ponctuelles affectant une région basique de sa région C-terminale.

2. Lentivirus selon la revendication 1, **caractérisé en ce que** ledit lentivirus est choisi parmi VIH-1, VIH-2, SIV, FIV, EIAV, BIV, VISNA et CAEV.

3. Lentivirus VIH-1 selon la revendication 2, **caractérisé en ce que** la mutation ponctuelle affectant la région basique L de sa région C-terminale est une substitution de l'acide aminé 186.

4. Lentivirus VIH-1 selon la revendication 2, **caractérisé en ce que** les mutations ponctuelles affectant la région basique Q de sa région C-terminale sont les mutations Q214L et/ou Q216L.

5. Lentivirus VIH-1 selon la revendication 2, **caractérisé en ce que** la mutation ponctuelle est une substitution du motif ₂₆₂RRK par le motif AAH.

6. Lentivirus selon la revendication 2, **caractérisé en ce que** l'élément d'export nucléaire de l'ARN comprend l'élément de réponse à REV (séquence RRE) du VIH-1.

7. Lentivirus selon l'une quelconque des revendications précédentes, **caractérisé en ce que** la séquence favorisant l'import nucléaire de l'ARN est le cPPT CTS.

8. Lentivirus selon l'une quelconque des revendications précédentes, **caractérisé en ce que** le transgène est un acide nucléique catalytique (interférant, antisens, ribozyme), un acide nucléique suicide ou un acide nucléique codant un polypeptide biologiquement actif, par exemple un facteur de croissance, un facteur trophique, une hormone, une cytokine, un anticorps, un récepteur, un facteur de différentiation, un facteur de stimulation des colonies, un agent anticancéreux, une toxine, un enzyme, un neurotransmetteur ou son précurseur.

9. Procédé de préparation d'un lentivirus selon l'une quelconque des revendications 1 à 8, **caractérisé en ce qu'**il comprend la transfection d'une cellule à l'aide d'un système vectoriel permettant l'obtention d'un lentivirus non intégratif et non réplicatif comprenant :
a. un plasmide de transcomplémentation, dépourvu de signal d'encapsidation psi et comprenant une séquence gag lentivirale et une séquence pol lentivirale mutée codant une intégrase non fonctionnelle pour l'intégration, la mutation dans la séquence pol du plasmide de transcomplémentation consistant en une ou plusieurs mutations ponctuelles affectant une région basique de la région C-terminale de l'intégrase, ledit plasmide étant éventuellement délété des gènes accessoires vif, nef, vpu et/ou vpr,
b. un plasmide d'enveloppe comportant une séquence Promoteur-env-PolyA, et
c. un plasmide vecteur lentiviral comportant un génome recombinant comprenant, entre les séquences LTR 5' et 3' lentivirales, une séquence psi d'encapsidation lentivirale, un élément d'export nucléaire de l'ARN, un transgène et éventuellement un promoteur et/ou une séquence favorisant l'import nucléaire de l'ARN, ainsi qu'une intégrase mutée empêchant l'intégration dudit génome dans le génome d'une cellule hôte, ledit vecteur étant dépourvu de toute séquence codante du lentivirus, et
la récupération des lentivirus produits.

10. Procédé de préparation d'un lentivirus selon l'une quelconque des revendications 1 à 8, **caractérisé en ce qu'**il comprend la transfection d'une cellule à l'aide d'un système vectoriel permettant l'obtention d'un lentivirus non intégratif et non réplicatif comprenant :
a. un plasmide de transcomplémentation, dépourvu de séquence d'encapsidation, de séquence codant pour une enveloppe, des gènes accessoires vif, nef, vpu et/ou vpr, et comprenant une séquence gag lentivirale et une séquence pol lentivirale mutée codant une intégrase non fonctionnelle pour l'intégration, la mutation dans la séquence pol consistant en une ou plusieurs mutations ponctuelles affectant une région basique de la région C-terminale de l'intégrase,
b. un plasmide d'enveloppe, dépourvu de signal d'encapsidation psi, de séquences codant gag ou pol et de LTRs lentiviraux, comportant une séquence Promoteur-env-PolyA, et
c. un plasmide vecteur lentiviral comportant un acide nucléique recombinant comprenant, entre les séquences LTR 5' et 3' lentivirales, une séquence psi d'encapsidation lentivirale, la séquence RRE, un transgène et éventuellement un promoteur et/ou le cPPT CTS, ledit plasmide vecteur lentiviral étant dépourvu des gènes viraux codant pour les séquences gag, pol et env et des gènes accessoires (vif, nef, vpr et/ou vpu), et
la récupération des lentivirus produits.

11. Procédé selon la revendication 9 ou 10, **caractérisé en ce que** le plasmide de transcomplémentation comprend en outre un élément de régulation de la transcription choisi parmi WPRE, le 5'UTR de l'APP, le 3'UTR de TAU, une séquence insulatrice.

12. Procédé selon l'une quelconque des revendications 9 à 11, **caractérisé en ce que** la protéine d'enveloppe du plasmide d'enveloppe est choisie parmi VSV-G, une protéine d'enveloppe d'un virus du sérogroupe du virus de la Rage: Rabies (RAB), Duvenhague (DUV), European bat type 1 (EB-1), European bat type 2 (EB-2), Kotonkan (KOT), Lagos bat (LB), Obodhiang (OBD), Rochambeau (RBU), une protéine d'enveloppe d'un virus du sérogroupe du virus de Mokola (MOK) et toute composition chimérique de ces enveloppes.

13. Procédé selon l'une quelconque des revendications 9 à 12, **caractérisé en ce que** le promoteur présent dans le plasmide d'enveloppe et/ou le promoteur présent dans le plasmide vecteur sont identiques ou différents et cellulaires ou viraux.

14. Procédé selon la revendication 13, **caractérisé en ce que** le ou les promoteur(s) utilisé(s) est un promoteur cellulaire choisi parmi le promoteur PGK, Rho et EF1α.

15. Procédé selon la revendication 13, **caractérisé en ce que** le ou les promoteur(s) utilisé(s) est un promoteur viral choisi parmi le promoteur du gène CMV, TK, RSV LTR, et le promoteur U6 ou H1 de l'ARN polymérase III.

16. Procédé selon l'une quelconque des revendications 9 à 12 **caractérisé en ce que** le plasmide vecteur lentiviral est dépourvu de la région promotrice ou de la séquence enhancer U3 du LTR3'.

17. Utilisation d'un lentivirus non intégratif et non réplicatif comprenant (i) un génome recombinant, dépourvu de toute séquence lentivirale codante, comprenant, entre les séquences LTR 5' et 3' lentivirales, une séquence psi d'encapsidation lentivirale, un élément d'export nucléaire de l'ARN, un transgène et, éventuellement, un promoteur et/ou une séquence favorisant l'import nucléaire de l'ARN, ainsi que (ii) une intégrase mutée empêchant l'intégration dudit génome dans le génome d'une cellule hôte, ladite mutation de l'intégrase consistant en une ou plusieurs mutations ponctuelles affectant une région basique de sa région C-terminale, pour la préparation d'une composition destinée au traitement d'une maladie du système nerveux (y compris de la sphère oculaire) d'un sujet.

18. Lentivirus non intégratif et non réplicatif comprenant (i) un génome recombinant, dépourvu de toute séquence lentivirale codante, comprenant, entre les séquences LTR 5' et 3' lentivirales, une séquence psi d'encapsidation lentivirale, un élément d'export nucléaire de l'ARN, un transgène et, éventuellement, un promoteur et/ou une séquence favorisant l'import nucléaire de l'ARN, ainsi que (ii) une intégrase mutée empêchant l'intégration dudit génome dans le génome d'une cellule hôte, ladite mutation de l'intégrase consistant en une ou plusieurs mutations ponctuelles affectant une région basique de sa région C-terminale, pour traiter une maladie du système nerveux (y compris de la sphère oculaire) d'un sujet.

19. Utilisation selon la revendication 17 ou lentivirus selon la revendication 18, pour le traitement des maladies neurodégénératives (Maladie de Parkinson, Maladie de Huntington, Maladie d'Alzheimer, SLA, SMA, DMLA, etc.), des traumas du système nerveux central (trauma de la moelle épinière, attaque cérébrale, etc.), des maladies métaboliques affectant le système nerveux (mucopolysaccharidoses, Charcot-Marie, etc.) ou des maladies affectant la sphère oculaire (rétinite pigmentaire, glaucome, etc.).

20. Composition pharmaceutique comprenant un lentivirus selon l'une des revendications 1 à 8 et un excipient acceptable sur le plan pharmaceutique.

## Claims

1. A recombinant non-replicative and non-integrative lentivirus comprising (i) a recombinant genome, deleted from all of the lentiviral encoding sequences, comprising, between the lentiviral LTR 5' and 3' sequences, a lentiviral encapsidation psi sequence, a RNA nuclear export element, a transgene and, optionally, a promoter and/or a sequence favouring the nuclear import of RNA, as well as (ii) a mutated integrase preventing the integration of said genome into the genome of a host cell, said integrase mutation consisting in one or more point mutations affecting a basic region of its C-terminal region.

2. The lentivirus according to claim 1, **characterized in that** said lentivirus is chosen from HIV-1, HIV-2, SIV, FIV, EIAV, BIV, VISNA and CAEV.

3. The VIH-1 lentivirus according to claim 2, **characterized in that** the point mutation affecting the L basic region of its C-terminal region is a substitution of amino acid 186.

4. The VIH-1 lentivirus according to claim 2, **characterized in that** the point mutations affecting the Q basic region of its C-terminal region are Q214L and/or Q216L mutations.

5. The VIH-1 lentivirus according to claim 2, **characterized in that** the point mutation is a substitution of the ₂₆₂RRK motif by the AAH motif.

6. The VIH-1 lentivirus according to claim 2, **characterized in that** the RNA nuclear export element comprises the REV responsive element (RRE sequence) of HIV-1.

7. The lentivirus according to anyone of the preceding claims, **characterized in that** the sequence favouring the nuclear import of RNA is cPPT CTS.

8. The lentivirus according to anyone of the preceding claims, **characterized in that** the transgene is a catalytic nucleic acid (interferent, antisense, ribozyme), a suicide nucleic acid or a nucleic acid encoding a biologically active polypeptide, for example a growth factor, a trophic factor, a hormone, a cytokine, an antibody, a receptor, a differentiation factor, a colony stimulation factor, an anticancer agent, a toxin, an enzyme, a neurotransmitter or its precursor.

9. A method of preparing a lentivirus according to anyone of the preceding claims 1 to 8, **characterized in that** it comprises the transfection of a cell with the aid of a vector system making it possible to obtain a non-integrative and non-replicative lentivirus, comprising:
a. a transcomplementation plasmid, lacking any psi encapsidation signal and comprising a gag lentiviral sequence and a mutated pol lentiviral sequence encoding a non-functional integrase for the integration, the mutation in the pol sequence of the transcomplementation plasmid consisting in one or more point mutations affecting a basic region of the integrase C-terminal region, said plasmid being optionally deleted from accessory genes vif, nef, vpu, and/or vpr,
b. an envelope plasmid including a Promoter-env-PolyA sequence, and
c. a lentiviral plasmid vector including a recombinant genome comprising, between the lentiviral LTR 5' and 3' sequences, a lentivirale encapsidation psi sequence, a RNA nuclear export element, a transgene and optionally a promoter and/or a sequence favouring the nuclear import of RNA, as well as a mutated integrase preventing the integration of said genome into the genome of a host cell, said vector being deleted from all of the lentiviral encoding sequences, and
recovery of lentivirus produced.

10. A method of preparing a lentivirus according to anyone of claims 1 to 8, **characterized in that** it comprises the transfection of a cell with the aid of a vector system making it possible to obtain a non-integrative and non-replicative lentivirus comprising:
a. a transcomplementation plasmid, lacking any encapsidation sequence, sequence encoding for an envelope, accessory genes vif, nef, vpu and/or vpr, and comprising a gag lentiviral sequence and a mutated pol lentiviral sequence encoding a non-functional integrase for the integration, the mutation in the pol sequence consisting in one or more point mutations affecting a basic region of the integrase C-terminal region.
b. an envelope plasmid, lacking any psi encapsidation signal, gag or pol encoding sequences and lentiviral LTRs, including a Promoter-env-PolyA sequence, and
c. a lentiviral plasmid vector including a recombinant nucleic acid comprising, between the lentiviral LTR 5' and 3' sequences, a lentiviral encapsidation psi sequence, the RRE sequence, a transgene and optionally a promoter and/or the cPPT CTS, said lentiviral plasmid vector being deleted from the viral genes encoding gag, pol and env sequences and accessory genes (vif, nef, vpr and/or vpu), and
recovery of lentiviruses produced.

11. A method according to claim 9 or 10, **characterized in that** the transcomplementation plasmid further comprises a transcription regulation element chosen from WPRE, the APP 5' UTR, the TAU 3'UTR, an insulating sequence.

12. A method according to anyone of claims 9 to 11, **characterized in that** the envelope protein of the envelope plasmid is chosen from VSV-G, an envelope protein of a virus from the serogroup of the Rabies virus: Rabies (RAB), Duvenhague (DUV), European bat type 1 (EB-1), European bat type 2 (EB-2), Kotonkan (KOT), Lagos bat (LB), Obodhiang (OBD), Rochambeau (RBU); an envelope protein of a virus from the serogroup of the Moloka virus (MOK) and any chimeric composition of these envelopes.

13. A method according to anyone of claims 9 to 12, **characterized in that** the promoter present in the envelope plasmid and/or the promoter present in the vector plasmid are identical or different and cellular or viral.

14. A method according to claim 13, **characterized in that** the promoter(s) used is a cellular promoter chosen from the PGK, Rho and EF1α promoter.

15. A method according to claim 13, **characterized in that** the promoter(s) used is a viral promoter chosen from the promoter of the CMV, TK, RSV LTR gene, and the U6 or H1 promoter of RNA polymerase III.

16. A method according to anyone of claims 9 to 12 **characterized in that** the lentiviral plasmid vector is lacking the promoter region or the LTR3' U3 enhancer sequence.

17. Use of a non-integrative and non-replicative lentivirus comprising (i) a recombinant genome, deleted from all of the lentiviral encoding sequences, comprising, between the lentiviral LTR 5' and 3' sequences, a lentiviral encapsidation psi sequence, a RNA nuclear export element, a transgene and, optionally, a promoter and/or a sequence favouring the nuclear import of RNA, as well as (ii) a mutated integrase preventing the integration of said genome into the genome of a host cell, said integrase mutation consisting in one or more point mutations affecting a basic region of its C-terminal region, for the preparation of a composition intended for the treatment of a disease of the nervous system (including of the ocular sphere) of a subject.

18. A non-integrative and non-replicative lentivirus comprising (i) a recombinant genome, deleted from all of the lentiviral encoding sequences, comprising, between the lentiviral LTR 5' and 3' sequences, a lentiviral encapsidation psi sequence, a RNA nuclear export element, a transgene, and optionally, a promoter and/or a sequence favouring the nuclear import of RNA, as well as (ii) a mutated integrase preventing the integration of said genome into the genome of a host cell, said integrase mutation consisting in one or more point mutations affecting a basic region of its C-terminal region, for treating a disease of the nervous system (including of the ocular sphere) of a subject.

19. The use according to claim 17 or the lentivirus according to claim 18, for the treatment of neurodegenerative diseases (Parkinson's disease, Huntingdon's disease, Alzheimer's disease, ALS, SMA, AMD, etc.), traumas of the central nervous system (trauma of the spinal cord, apoplexy, etc.), metabolic diseases affecting the nervous system (mucopolysaccharidoses, Charcot-Marie, etc.) or diseases affecting the ocular sphere (retinitis pigmentosa, glaucoma, etc.).

20. A pharmaceutical composition comprising a lentivirus according to anyone of claims I to 8 and a pharmaceutically acceptable excipient.

## Patentansprüche

1. Nicht integratives und nicht replikatives rekombinantes Lentivirus, umfassend (i) ein rekombinantes Genom, ohne irgendeine codierende lentivirale Sequenz, umfassend zwischen den lentiviralen 3'- und 5'-LTR-Sequenzen eine lentivirale Verpackungssequenz psi, ein RNA-Kernexportelement, ein Transgen und gegebenenfalls einen Promotor und/oder eine Sequenz, die den RNA-Kernimport fördert, sowie (ii) eine mutierte Integrase, die die Integration besagten Genoms in das Genom einer Wirtszelle verhindert, wobei besagte Mutation der Integrase aus einer oder mehreren Punktmutationen besteht, die eine Basenregion ihrer C-terminalen Region betreffen.

2. Lentivirus gemäß Anspruch 1, **dadurch gekennzeichnet, dass** besagtes Lentivirus aus HIV-1, HIV-2, SIV, FIV, EIAV, BIV, VISNA und CAEV ausgewählt ist.

3. HIV-1 Lentivirus gemäß Anspruch 2, **dadurch gekennzeichnet, dass** die Punktmutation, die die Basenregion L der C-terminalen Region betrifft, eine Substitution der Aminosäure 186 ist.

4. HIV-1 Lentivirus gemäß Anspruch 2, **dadurch gekennzeichnet, dass** die Punktmutationen, die die Basenregion Q der C-terminalen Region betreffen, die Mutationen Q214L und/oder Q216L sind.

5. HIV-1 Lentivirus gemäß Anspruch 2, **dadurch gekennzeichnet, dass** die Punktmutation eine Substitution des ₂₆₂RRK-Motivs durch das AAH-Motiv ist.

6. Lentivirus gemäß Anspruch 2, **dadurch gekennzeichnet, dass** das RNA-Kernexportelement ein REV-Antwortelement (RRE-Sequenz) von HIV-1 umfasst.

7. Lentivirus gemäß einem der vorhergehenden Ansprüche, **dadurch gekennzeichnet, dass** die Sequenz, die den RNA-Kernimport fördert, die cPPT CTS ist.

8. Lentivirus gemäß einem der vorhergehenden Ansprüche, **dadurch gekennzeichnet, dass** das Transgen eine katalytische Nukleinsäure (interferierende, antisense, Ribozym), eine Selbstmord-Nukleinsäure oder eine Nukleinsäure ist, die ein biologisch aktives Polypeptid codiert, zum Beispiel einen Wachstumsfaktor, einen trophischen Faktor, ein Hormon, ein Cytokin, einen Antikörper, einen Rezeptor, einen Differenzierungsfaktor, einen Kolonie stimulierenden Faktor, ein Antikrebsmittel, ein Toxin, ein Enzym, einen Neurotransmitter oder seinen Vorläufer.

9. Verfahren zur Herstellung eines Lentivirus gemäß einem der Ansprüche 1 bis 8, **dadurch gekennzeichnet, dass** das Verfahren die Transfektion einer Zelle mithilfe eines Vektorsystems umfasst, das den Erhalt eines nicht replikativen und nicht integrativen Lentivirus erlaubt, umfassend:
a) ein trans-komplementierendes Plasmid ohne Verpackungssignal psi und umfassend eine lentivirale gag-Sequenz und eine mutierte lentivirale pol-Sequenz, die eine nicht zur Integration fähige Integrase codiert, wobei die Mutation in der pol-Sequenz des transkomplementierenden Plasmids aus einer oder mehreren Punktmutationen besteht, die eine Basenregion der C-terminalen Region der Integrase betreffen, wobei in besagtem Plasmid gegebenenfalls die akzessorischen Gene vif, nef, vpu und/oder vpr deletiert sind,
b) ein Hüllplasmid, umfassend eine Promotor-env-PolyA-Sequenz, und
c) einen lentiviralen Plasmidvektor, umfassend ein rekombinantes Genom, umfassend zwischen den lentiviralen 3'- und 5'-LTR-Sequenzen eine lentivirale Verpackungssequenz psi, ein RNA-Kernexportelement, ein Transgen und gegebenenfalls einen Promotor und/oder eine Sequenz, die den RNA-Kernimport fördert, sowie eine mutierte Integrase, die die Integration besagten Genoms in das Genom einer Wirtszelle verhindert, wobei besagter Vektor ohne irgendeine codierende Sequenz des Lentivirus ist, und
die Wiederfindung der Lentivirus-Produkte.

10. Verfahren zur Herstellung eines Lentivirus gemäß einem der Ansprüche 1 bis 8, **dadurch gekennzeichnet, dass** das Verfahren die Transfektion einer Zelle mithilfe eines Vektorsystems umfasst, das den Erhalt eines nicht replikativen und nicht integrativen Lentivirus erlaubt, umfassend:
a) ein trans-komplementierendes Plasmid ohne Verpackungssequenz, für eine Hülle codierende Sequenz, die akzessorischen Gene vif, nef, vpu und/oder vpr und umfassend eine lentivirale gag-Sequenz und eine mutierte lentivirale pol-Sequenz, die eine nicht zur Integration fähige Integrase codiert, wobei die Mutation in der pol-Sequenz aus einer oder mehreren Punktmutationen besteht, die eine Basenregion der C-terminalen Region der Integrase betreffen,
b) ein Hüllplasmid, ohne Verpackungssignal psi, gag oder pol codierende Sequenzen und lentivirale LTRs, umfassend eine Promotor-env-PolyA-Sequenz, und
c) einen lentiviralen Plasmidvektor, umfassend eine rekombinante Nukleinsäure, umfassend zwischen den lentiviralen 3'- und 5'-LTR-Sequenzen eine lentivirale Verpackungssequenz psi, die RRE-Sequenz, ein Transgen und gegebenenfalls einen Promotor und/oder die cPPT CTS, wobei besagter lentiviraler Plasmidvektor ohne die viralen Gene, die für die gag-, pol- und env-Sequenzen codieren, und die akzessorischen Gene (vif, nef, vpr und/oder vpu) ist, und
die Wiederfindung der Lentivirus-Produkte.

11. Verfahren gemäß Anspruch 9 oder 10, **dadurch** gekenntzeichnet, dass das transkomplementierende Plasmid außerdem ein Element zur Regulation der Transkription umfasst, das aus WPRE, 5`-UTR von APP, 3'-UTR von TAU, einer isolierenden Sequenz ausgewählt ist.

12. Verfahren gemäß einem der Ansprüche 9 bis 11, **dadurch gekennzeichnet, dass** das Hüllprotein des Hüllplasmids aus VSV-G, einem Hüllprotein eines Virus der Serogruppe des Rabiesvirus: Rabies (RAB), Duvenhage (DUV), European bat type 1 (EB-1), European bat type 2 (EB-2), Kotonkan (KOT), Lagos bat (LB), Obodhiang (OBD), Rochambeau (RBU), einem Hüllprotein eines Virus der Serogruppe des Mokola-Virus (MOK) und einer beliebigen chimären Zusammensetzung dieser Hüllen ausgewählt ist.

13. Verfahren gemäß einem der Ansprüche 9 bis 12, **dadurch gekennzeichnet, dass** der im Hüllplasmid vorliegende Promotor und/oder der im Plasmidvektor vorliegende Promotor identisch: oder verschieden und zellulär oder viral sind.

14. Verfahren gemäß Anspruch 13, **dadurch gekennzeichnet, dass** der oder die eingesetzte(n) Promotor(en) ein zellulärer Promotor ist, der aus dem PGK-, Rho- und EF1α-Promotor ausgewählt ist.

15. Verfahren gemäß Anspruch 13, **dadurch gekennzeichnet, dass** der oder die eingesetzte(n) Promotor(en) ein viraler Promotor ist, der aus dem Promotor des CMV-, TK-, RSV LTR-Gens und dem U6- oder H1-Promotor der RNA-Polymerase III ausgewählt ist.

16. Verfahren gemäß einem der Ansprüche 9 bis 12, **dadurch gekennzeichnet, dass** der lentivirale Plasmidvektor ohne die Promotorregion oder die U3-Enhancer-Sequenz der 3`-LTR ist.

17. Verwendung eines nicht replikativen und nicht integrativen Lentivirus, umfassend (i) ein rekombinantes Genom, ohne irgendeine codierende lentivirale Sequenz, umfassend zwischen den lentiviralen 3`- und 5'-LTR-Sequenzen eine lentivirale Verpackungssequenz psi, ein RNA-Kernexportelement, ein Transgen und gegebenenfalls einen Promotor und/oder eine Sequenz, die den RNA-Kernimport fördert, sowie (ii) eine mutierte Integrase, die die Integration besagten Genom in das Genom einer Wirtszelle verhindert, wobei besagte Mutation der Integrase aus einer oder mehreren Punktmutationen besteht, die eine Basenregion ihrer C-terminalen Region betreffen, zur Herstellung einer Zusammensetzung für die Behandlung einer Erkrankung des Nervensystems (einschließlich Auge) einer Person.

18. Nicht replikatives und nicht integratives Lentivirus, umfassend (i) ein rekombinantes Genom, ohne irgendeine codierende lentivirale Sequenz, umfassend zwischen den lentiviralen 3`- und 5'-LTR-Sequenzen eine lentivirale Verpackungssequenz psi, ein RNA-Kernexportelement, ein Transgen und gegebenenfalls einen Promotor und/oder eine Sequenz, die den RNA-Kernimport fördert, sowie (ii) eine mutierte Integrase, die die Integration besagten Genoms in das Genom einer Wirtszelle verhindert, wobei besagte Mutation der Integrase aus einer oder mehreren Punktmutationen besteht, die eine Basenregion ihrer C-terminalen Region betreffen, um eine Erkrankung des Nervensystems (einschließlich Auge) einer Person zu behandeln.

19. Verwendung gemäß Anspruch 17 oder Lentivirus gemäß Anspruch 18 zur Behandlung von neurodegenerativen Erkrankungen (Morbus Parkinson, Morbus Huntington, Morbus Alzheimer, ALS, SMA, altersbedingter Makuladegeneration etc.), Verletzungen des Zentralnervensystems (Rückenmarkverletzung, Schlaganfall etc.), Stoffwechselerkrankungen, die das Nervensystem befallen (Mucopolysaccharidosen, Charcot-Marie etc.), oder Krankheiten, die das Auge befallen (Retinitis pigmentosa, Glaukom etc.).

20. Pharmazeutische Zusammensetzung, umfassend ein Lentivirus gemäß einem der Ansprüche 1 bis 8 und einen pharmazeutisch verträglichen Arzneimittelträger.
